# EUROPEAN PATENT APPLICATION

(11) **EP 4 681 751 A2**
(43) Date of publication of application: **21.01.2026**
(21) Application number: 25220254.4
(22) Date of filing: 01.07.2019
(51) Int. Cl.: A61M 5/50

(54) **TIP CAP FOR A MEDICAL INJECTION DEVICE**

(62) Divisional of application: 19305897.1
(71) Applicant: Becton Dickinson France, 38800 Le Pont de Claix (FR)
(72) Inventor: EUVRARD, Nicolas, DURHAM, 27701 (US)
(74) Representative: Regimbeau

(57) **Abstract**

The invention relates to a tip cap (1) for a medical injection device, comprising:
- a tip connector (100) configured to engage a tip of the medical injection device;
- a closure cap (101) configured to sealingly close a distal opening of the tip, the closure cap being releasably connected to the tip connector (100);
said tip cap further comprising an RFID tag (2) comprising a chip (20) and an antenna (21), wherein the chip and the antenna are overmolded in at least one of the tip connector (100) and the closure cap (101), the chip (20) and the antenna (21) being electrically connected by a bridge (22) extending through the frangible portion (100c) of the tip connector (100) and configured to break under a relative movement applied to the closure cap (101) and the tip connector (100) in order to release the closure cap from the tip connector.

## Description

### TECHNICAL FIELD

The invention relates to a tip cap for a medical injection device and to a process for manufacturing such a tip cap.

### TECHNICAL BACKGROUND

Medical injection devices such as syringes typically include a container for containing a pharmaceutical composition, said container having an end piece in a form of a longitudinal tip defining a fluid path through which the pharmaceutical composition is expelled. Medical injection devices may be prefilled with pharmaceutical compositions, which may be sensitive.

There is an increasing need for individual traceability of the medical injection devices, and typically of the medical containers, from the manufacturing process until at least the final use of said medical containers, typically the pharmaceutical composition injection. Indeed, it is desirable to enable traceability of such medical devices, in order to record all events that happened during the manufacturing process, the filling process and/or the use of the medical injection device. Medical injection devices are usually not processed individually but may be carried or packed with a plurality of similar medical injection devices, the traceability should ensure that the status of the medical containers may be checked at any time during the life cycle of the medical injection devices, and that this checking of the status may be implemented without unpacking the medical injection devices.

Besides, there is a need to make sure that a medical injection device contains the expected pharmaceutical composition and may not be refilled with another pharmaceutical composition than the original one. Moreover, there is a need to ensure, that when a medical injection device is up to be used, said medical injection device has not been opened before. Accordingly, it may be desirable to provide such medical injection devices with tamper-evidence means.

To date, there does not exist any practical solution enabling both traceability and tamper-evidence of medical injection devices.

### SUMMARY OF THE DISCLOSURE

A goal of the invention is to provide a medical injection device comprising traceability and tamper-evidence means.

This goal may be achieved by providing a tip cap for a medical injection device, comprising:
- a tip connector configured to engage a tip of the medical injection device;
- a closure cap configured to sealingly close a distal opening of the tip, the closure cap being releasably connected to the tip connector;
said tip cap being characterized in that it comprises an RFID tag comprising a chip and an antenna, wherein the chip and the antenna are overmolded in at least one of the tip connector and the closure cap, the chip and the antenna being electrically connected by a bridge configured to break under a relative movement applied to the closure cap and the tip connector in order to release the closure cap from the tip connector.

The tip cap for a medical injection device may comprise:
- a tip connector configured to engage a tip of the medical injection device, comprising a main body and an end portion connected to the main body by a frangible portion;
- a closure cap configured to sealingly close a distal opening of the tip, the closure cap being releasably connected to the tip connector by mutually engaging threaded portions;
the tip cap further comprising an RFID tag integral with the tip connector and comprising a chip and an antenna,
wherein the chip is overmolded in one of the main body and the end portion and the antenna is overmolded in the other one of the main body and the end portion, the chip and the antenna being electrically connected by a bridge extending through the frangible portion of the tip connector, the bridge being configured to break under a relative movement applied to the closure cap and the tip connector in order to release the closure cap from the tip connector.

The RFID tag provides both traceability of the medical container until opening of the closure cap, and tamper-evidence since it is deactivated upon opening of the closure cap by breakage of the electrical connection between the chip and the antenna. Besides, the overmolding process used to integrate the RFID tag to the tip cap minimizes the number of assembling steps and the manufacturing cost of the tip cap.

In the present text, the terms "distal" and "proximal" are relative terms defining a region which is further, respectively closer - along an axis of injection of a pharmaceutical composition, to a user carrying the medical injection device in order to inject the pharmaceutical composition into a patient's body. For example, when the medical injection device is a syringe comprising a flange configured to be held between the user's fingers and a tip coupled to a needle configured to prick the patient's tissues and inject the pharmaceutical composition, the flange is considered to be proximal and the tip is considered to be distal.

According to advantageous but not limiting embodiments, possibly combined:
- the closure cap comprises a sealing septum configured to abut the tip of the medical injection device;
- the antenna presents a ring shape extending about a longitudinal axis of the tip cap;
- the bridge is parallel to the longitudinal axis of the tip cap;
- the bridge is orthogonal to the longitudinal axis of the tip cap;
- the tip connector and the closure cap comprise mutually engaging protrusions and notches configured to rotationally secure the end portion of the tip connector to the closure cap, the frangible portion being configured to break under a relative rotational movement applied to the closure cap and the tip connector in order to release the closure cap from the main body of the tip connector;

Another object is a medical injection device comprising a barrel and a tip comprising a distal opening, and a tip cap as described above mounted on said tip.

Another object is a process for manufacturing a tip cap comprising a tip connector and a closure cap releasably connected to the tip connector, comprising:
i) providing a RFID tag comprising a chip and an antenna, the chip and the antenna being electrically connected by a bridge;
ii) placing the RFID tag in a mold adapted for injection molding of the tip connector and/or the closure cap,
iii) injecting at least one plastic material into the mold so as to form the tip connector and/or the closure cap by overmolding the chip and the antenna.

According to an embodiment of said process:
- in step ii), the RFID tag is placed in a mold adapted for injection molding of the tip connector,
- in step iii), the plastic material is injected into the mold by using a first insert so as to form at least a part of the tip connector and by overmolding the chip and the antenna, the chip and the antenna presenting a flushed surface;
and the process further comprises the following steps:
iv) injecting the same plastic material as in step iii) by using a second insert and obtaining the tip connector in which the RFID tag is overmolded;
v) molding the closure cap; and
vi) releasably assembling the closure cap to the tip connector in order to obtain the tip cap.

The process can comprise:
i) providing a RFID tag comprising a chip and an antenna, the chip and the antenna being electrically connected by a bridge;
ii) placing the RFID tag in a mold adapted for injection molding of the tip connector;
iii) injecting at least one plastic material into the mold by using a first insert so as to form at least a part of the tip connector and by overmolding the chip and the antenna, the chip and the antenna presenting a flushed surface;
iv) injecting the same plastic material as in step iii) by using a second insert and obtaining the tip connector in which the RFID tag is overmolded;
v) molding the closure cap; and
vi) releasably assembling the closure cap to the tip connector in order to obtain the tip cap.

According to another embodiment of said process:
- in step ii), the RFID tag is placed in a mold adapted for two-shot injection molding of the tip connector and the closure cap,
- in step iii), a first plastic material is injected into the mold so as to form at least the tip connector, the chip of the RFID tag being overmolded into the tip connector;
and the process further comprises the following steps:
iv) injecting into the mold a second plastic material which is chemically not bonding with the first plastic material so as to form the closure cap, the chip and the antenna being disposed at the interface of the tip connector and the closure cap; and
v) obtaining the releasably assembled tip cap.

### BRIEF DESCRIPTION OF THE DRAWINGS

Further features, embodiments and advantages of the invention will be apparent from the following detailed description, based on the appended drawings wherein:
- FIG. 1 is a sectional view of a tip cap according to a first embodiment;
- FIG. 2 is a perspective view of a closure cap of the tip cap according to the first embodiment;
- FIG. 3 is a perspective view of a tip connector of the tip cap according to the first embodiment;
- FIG.4 is a perspective view of an RFID tag of the tip cap according to the first embodiment;
- FIGS. 5A and 5B are a view of the relative position of the closure cap and the tip connector respectively during screwing and after complete screwing of the closure cap onto the tip cap according to the first embodiment;
- FIG. 6 is a sectional view of the tip cap according to the first embodiment after unscrewing the closure cap from the tip connector;
- FIG. 7 is a sectional view of the tip cap of FIG. 6 after screwing again the closure cap onto the tip connector;
- FIG. 8 is a perspective view of a medical injection device with a tip cap according to a second embodiment;
- FIG. 9 is an enlarged view of the tip cap of FIG. 8;
- FIG. 10 is an enlarged view of the RFID tag overmolded in the tip cap of FIG. 8;
- FIG. 11 is a partial view of the tip cap according to the second embodiment, showing the tip connector and the RFID tag;
- FIG. 12 is a partial view of the tip cap according to the second embodiment, showing the closure cap and the RFID tag.

### DETAILED DESCRIPTION OF EMBODIMENTS

The tip cap of the invention is configured to be mounted on a medical injection device. Said medical injection device may comprise a barrel extending about a longitudinal axis and a distal tip extending from a distal end of the barrel. The distal tip of the medical injection device comprises a longitudinal through hole for expelling a pharmaceutical composition which may be contained in the barrel.

The tip may be covered by a tip cap. Said tip cap comprises a tip connector configured to engage the tip and a closure cap configured to sealingly close a distal opening of the tip. The closure cap may include a sealing septum abutting the tip of the medical injection device, and especially the distal end of the tip of said medical injection device.

The closure cap may be releasably connected to the tip connector. Before use of the medical injection device, the closure cap may be mounted onto the tip connector to protect the distal tip from shocks and external contamination. When the medical injection device is to be used for injection of a pharmaceutical composition to a patient, the closure cap is removed from the tip connector in order to expose the tip of the medical injection device. A needle may then be connected to the tip connector to perform the injection.

According to the invention, the tip cap includes a Radio-Frequency Identification tag (RFID tag).

In a manner known per se, the RFID tag comprises a chip and an antenna electrically connected by a bridge. Unique identification data about the medical container and data relating to the processing of the medical container may be recorded in the chip. Said data may be read by a suitable RFID reader. However, if any part of the RFID tag is broken, the RFID tag losses its integrity and can no longer be read by the RFID reader.

In order to provide a tamper-evidence function of the tip cap, the chip and the antenna are overmolded in at least one of the tip connector and the closure cap. The chip and the antenna are arranged with respect to the tip connector and closure cap so that during a relative movement applied to the closure cap and the tip connector to release the closure cap from the tip connector, the electrically connected bridge is configure to break. More specifically, one part of the RFID tag (e.g. the chip) remains fixed to the tip connector while another part of the RFIF tag (e.g. the antenna) remains fixed to the closure cap. This movement results in the breakage of a part of the RFID tag (e.g. the bridge), which causes deactivation of the RFID tag.

In this way, when the closure cap has been opened, a clear indication of the tip cap status (already open or intact) is provided by attempting to read the RFID tag. Besides, as long as the tip cap is not opened, the tip cap features a detectable unique device identifier enabling product traceability between the assembly at medical injection device manufacturer's until drug container secondary packaging at pharmaceutical lab level, and ideally until closure cap removal.

Advantageously, said relative movement may be a rotational movement, a translational movement, or a combination of rotational and translational movements.

In preferred embodiments, the relative movement may be an unscrewing movement. To that end, the tip connector and the closure cap comprise mutually engaging threaded portions.

The antenna may advantageously present a ring shape extending about a longitudinal axis of the tip cap, which coincides with the longitudinal axis of the medical container.

According to an embodiment, the bridge may be parallel to said longitudinal axis.

Alternatively, the bridge may be orthogonal to the longitudinal axis of the tip cap.

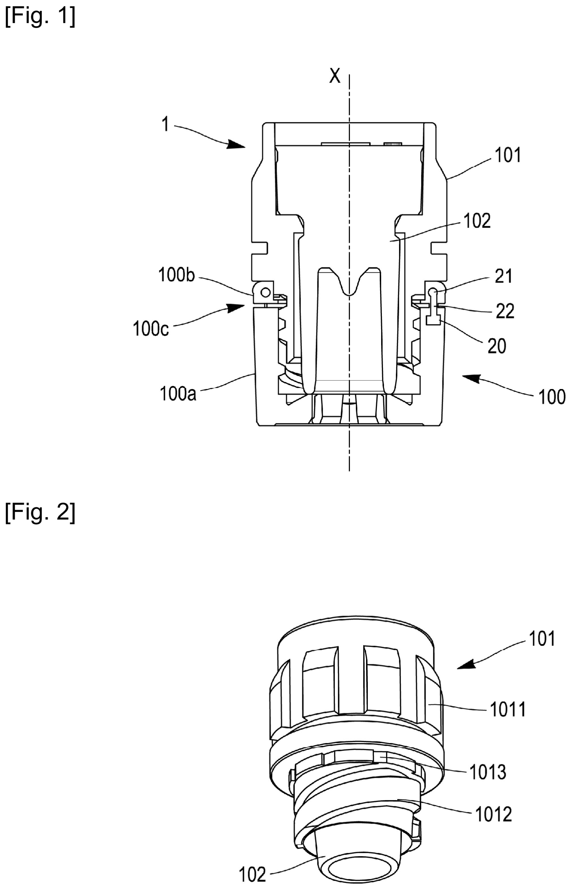

FIGS. 1 to 7 relate to a first embodiment of the tip cap.

FIG. 1 is a sectional view of the tip cap before use of the medical injection device.

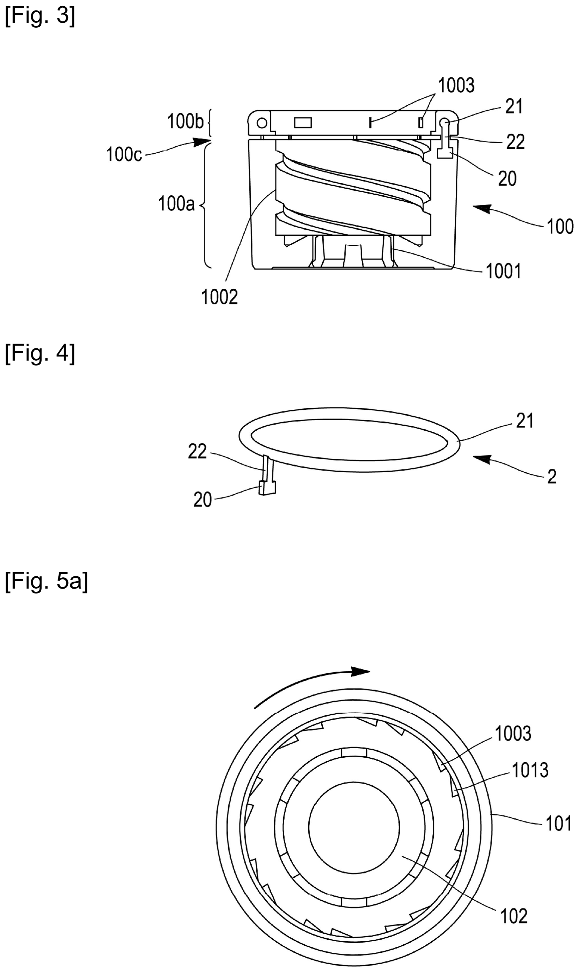

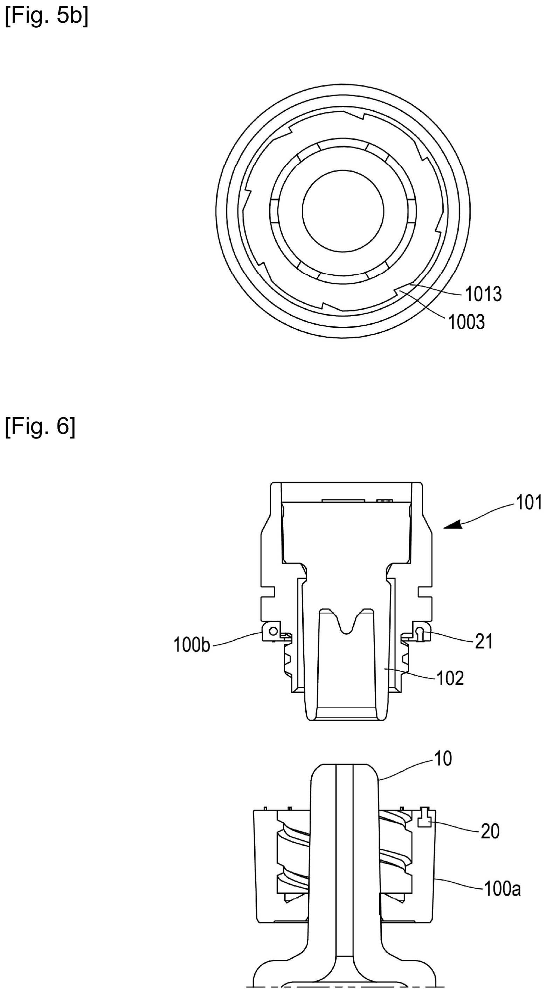

The tip cap comprises a tip connector 100 configured to be attached to a tip of the medical injection device (shown as 10 in FIGS. 6-7), and a closure cap 101 releasably screwed onto the tip connector 100 to sealingly close the distal opening of the tip. To that end, the tip connector 100 and the closure cap 101 comprise mutually engaging threaded portions 1002, 1012 (see FIGS. 2 and 3). When the medical injection device is to be used, the closure cap 101 is unscrewed from the tip connector 100 to provide access to the tip whereas the tip connector remains fixed to the tip. A needle may thus be connected to the tip connector via the threaded portion 1002. The tip cap extends around a longitudinal axis X of the medical injection device. In the present text, the term "axially" refers to axis X.

As better seen in FIG. 3, the tip connector 100 comprises a proximal ring portion 1001 configured to be mounted onto the tip of the medical injection device. According to an embodiment, the tip connector is a Luer lock adapter (LLA). The inner surface of the ring portion 1001 may have a frustoconical shape enabling frictional engagement with the tip of the medical injection device.

The tip connector 100 further comprises a distal female threaded portion 1002 configured to cooperate with the proximal male threaded portion 1012 of the closure cap (see FIG. 2).

As shown on FIG. 2, the closure cap 101 comprises a distal handling portion 1011. The handling portion advantageously comprises an outer surface configured to be easily held between a user's fingers, in particular for screwing or unscrewing the closure cap onto the tip connector. Said outer surface may be grooved in order to avoid slipping of the fingers during such a screwing or unscrewing motion.

The closure cap 101 may be made of a rigid material such as plastic. The closure cap 101 advantageously comprises a sealing septum 102, made of a flexible material such as rubber or thermoplastic elastomer (TPE). The inner cap is fixed to the closure cap, e.g. by frictional engagement or clipping. When the tip cap is mounted onto the medical injection device, the proximal end of the inner flexible cap is in contact with the distal end of the tip to create a tight seal protecting the content of the medical injection device from external contamination.

The tip connector comprises a proximal main body 100a and a distal end portion 100b connected to the main body by a frangible portion 100c. The frangible portion is configured to break under a relative rotational movement about axis X applied to the end portion 100b and the main body 100a. In other embodiments, the frangible portion is configured to break under a relative translational movement or a combination of relative rotational and translational movement about axis X applied to the end portion 100b and the main body 100a. The frangible portion 100c may comprise a plurality of links distributed over the circumference of the tip connector. Said links have a relatively small cross section and extend axially between the main body 100a and the end portion 100b.

The tip connector 100 and the closure cap 101 comprise mutually engaging protrusions 1003 and notches 1013 configured to rotationally secure the end portion 100b of the tip connector to the closure cap 101.

As shown in FIG. 5A, during screwing the closure cap onto the tip connector (direction indicated by the arrow), the protrusions 1003 do not match the notches 1013.

When the screwing is complete (see FIG. 5B), each protrusion 1003 engages a respective notch 1013.

Each notch and corresponding protrusion have a non-symmetrical profile relative to a radius of the tip cap. In the screwing direction, the front side of each protrusion 1003 extends substantially radially, whereas the rear side of the protrusion 1003 extends obliquely. Otherwise said, the front side of the protrusion forms a steep step extending from the inner surface of the tip connector, whereas the rear side of the protrusions is connected to said inner surface via a gentle slope. The shape of the notches of the closure cap is complementary to the one of the protrusions.

This shape of the protrusions and notches allow screwing the closure cap onto the tip connector without exerting an excessive effort. However, in the unscrewing direction, the notches of the closure cap 101 apply a torque on the distal end portion 100b of the tip connector, thereby causing the the weak links forming the frangible portion 100c to break.

The tip connector comprises an RFID tag 2 (illustrated in perspective in FIG. 4). The RFID tag comprises an RFID chip 20 and an antenna 21, the RFID chip being coupled to the antenna by a bridge 22. The antenna 21 preferably presents an annular shape extending around the axis X, but may present any other suitable shape. Any breakage of one of the components of the RFID tag, in particular of the bridge 22, inactivates the RFID tag.

In this first embodiment according to the invention, the RFID tag is integrated, preferably fully integrated, in the tip connector 100 in such a way that the RFID chip 20 and the antenna 21 are in different portions of the tip connector with respect to the frangible portion 100b. For example, as illustrated in FIG. 3, the chip 20 is integrated in the main body 100a whereas the antenna is integrated in the end portion 100b, the bridge extending through the frangible portion 100c. In an alternative embodiment (not illustrated), the chip could be integrated in the end portion and the antenna in the main body of the tip connector.

When a relative rotational movement is applied to the tip connector and the closure cap in order to remove the closure cap from the tip connector, both the frangible portion 100c and the bridge 22 break, thereby breaking the electrical connexion between the chip and the antenna and inactivating the RFID tag.

As a result, as shown in FIG. 6, when the closure cap 101 is removed from the tip connector 100, the distal end portion 100b and the antenna 21 of the RFID tag remain attached to the closure cap 100 via the engagement of the notches and protrusions, whereas the chip 20 of the RFID tag remains attached to the main body 100a of the tip connector.

Even if the closure cap 101 is screwed again onto the main body 100a of the tip connector, the RFID tag remains inactive since the broken bridge no longer electrically connects the chip and the antenna. Thus, the RFID tag cannot be read anymore.

If a user wants to check whether the tip cap has been opened, he has to try to read the RFID tag with an appropriate RFID reader. If the RFID reader does not detect any tag, it means that the tip cap has been opened. On the contrary, if the RFID reader detects the tag, it means the tip cap is intact.

The tip cap may be manufactured as follows.

On the one hand, the closure cap 101 and sealing septum 102 may be fabricated by injection molding and assembled. Suitable materials for the closure cap 101 include polypropylene or any other rigid plastic material, and suitable materials for the sealing septum 102 include rubber and thermoplastic elastomer.

On the other hand, the tip connector with the RFID tag may be fabricated by an overmolding process.

To that end, the RFID tag is placed in a mold adapted for injection molding of the tip connector. The mold includes a first insert configured to hold the RFID tag in the intended position relative to the main body and distal end portion of the tip connector. In a first injection step, the plastic material is injected into the mold so as to partially overmold the chip and the antenna. At the end of this first injection step, the chip and the antenna present a flushed surface. Then, the part resulting from the first injection step is removed and placed in a second insert of the mold to define the shape of the remaining portion of the tip connector. In a second injection step, the same plastic material as in the first injection step is thus injected into the mold, thereby fully overmolding the RFID tag. Suitable materials for the tip connector include polypropylene or any other rigid plastic material.

The closure cap may then be releasably assembled to the tip connector by screwing until engagement of the protrusions of the end portion of the tip connector into the notches of the closure cap.

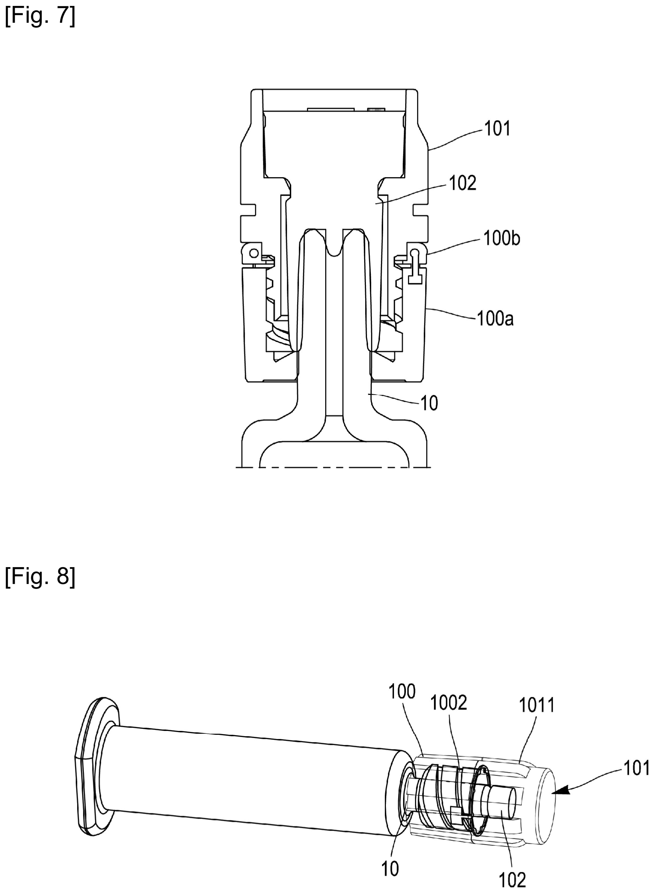

FIGS. 8 to 12 illustrate a second embodiment of the tip cap. The elements identified by the same reference numerals than in FIGS. 1-7 have the same function.

As in the first embodiment, the tip cap comprises a tip connector 100 configured to engage a tip 10 of the medical injection device, and a closure cap 101 releasably screwed to the tip connector and configured to sealingly close a distal opening of the tip 10.

To that end, the tip connector 100 and the closure cap 101 comprise mutually engaging threaded portions 1002. When the medical injection device is to be used, the closure cap 101 is unscrewed from the tip connector 100 to provide access to the tip whereas the tip connector remains fixed to the tip. A needle may thus be connected to the tip connector via the threaded portion 1002. The tip cap extends around a longitudinal axis X of the medical injection device.

The tip connector 100 comprises a proximal ring portion configured to be mounted onto the tip of the medical injection device. According to an embodiment, the tip connector is a Luer lock adapter (LLA). The inner surface of the ring portion may have a frustoconical shape enabling frictional engagement with the tip of the medical injection device.

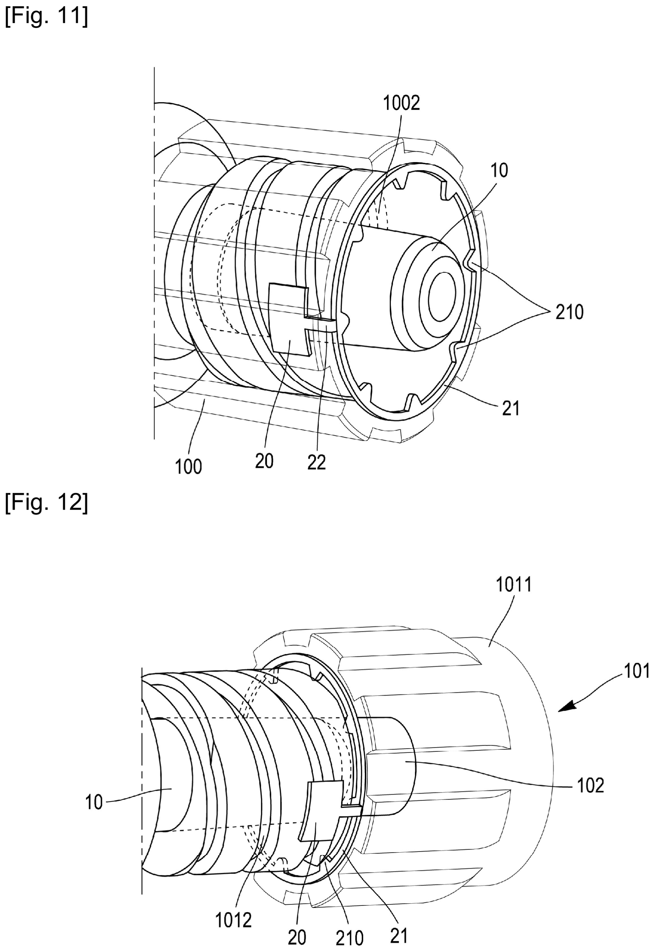

As better seen in FIGS. 11 and 12, the tip connector 100 further comprises a distal female threaded portion 1002 configured to cooperate with the proximal male threaded portion 1012 of the closure cap.

The closure cap 101 comprises a distal handling portion 1011. The handling portion advantageously comprises an outer surface configured to be easily held between a user's fingers, in particular for screwing or unscrewing the closure cap onto the tip connector. Said outer surface may be grooved in order to avoid slipping of the fingers during such a screwing or unscrewing motion.

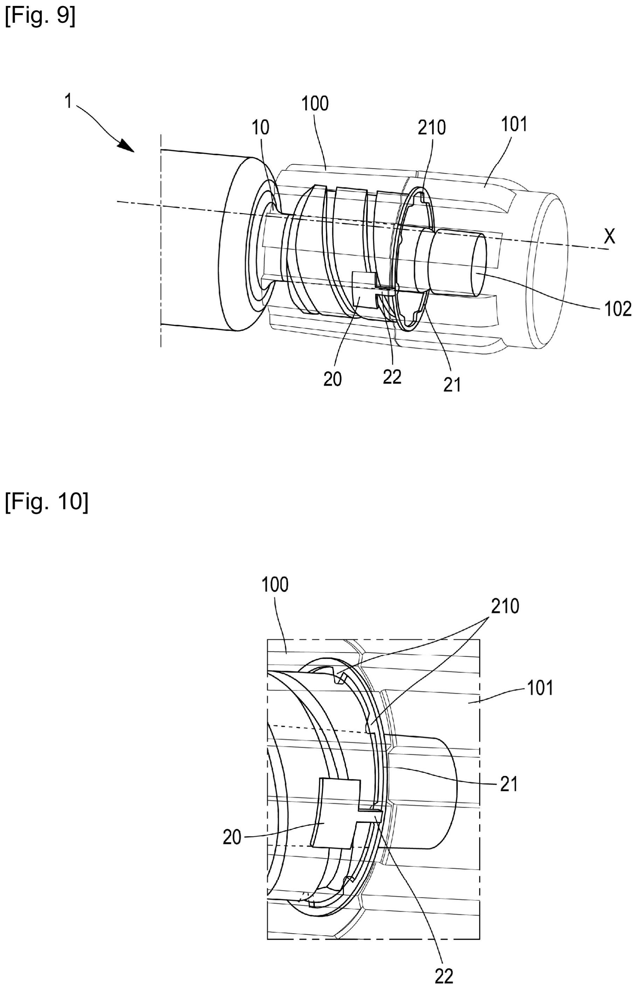

As seen on FIG. 9, the closure cap 101 includes a sealing septum 102 configured to be in contact with the distal end of the tip to create a tight seal protecting the content of the medical injection device from external contamination. In this embodiment, the sealing septum 102 is integral with the closure cap 101.

The tip connector 100 and the closure cap 101 are made from chemically not bonding materials. By "chemically not bonding materials" is meant a pair of materials which do not have chemical bonds between each other when a first material is molded over the second material. As a result, if a part made of the second material is overmolded into a part made of the first material, the parts are not bonded to each other and may be moved relative to one another.

According to an embodiment, the tip connector may be made of polycarbonate (PC) and the closure cap may be made of a thermoplastic elastomer (TPE). However, other pairs of chemically not bonding materials suitable for the application may be used.

The tip cap comprises an RFID tag 2. The RFID tag comprises an RFID chip 20 and an antenna 21, the RFID chip being coupled to the antenna by a bridge 22. The antenna 21 preferably presents an annular shape extending around the axis X, but may present any other suitable shape. Any breakage of one of the components of the RFID tag, in particular of the bridge 22, inactivates the RFID tag.

The RFID tag is integrated in the tip cap in such a way that the RFID chip 20 and the antenna 21 are in different portions of the tip cap. In the illustrated embodiment, the chip 20 is overmolded in the tip connector 100 and the antenna 21 is overmolded in the closure cap 101, the antenna being located at an interface between the tip connector and the closure cap. In an alternative embodiment (not illustrated), the chip could be integrated in the closure cap and the antenna in the tip connector, the antenna still being located at the interface between the tip connector and the closure cap.

The tip cap is thus made as a single piece with the tip connector and the closure cap overmolded onto the RFID tag. Due to the fact that the tip connector and the closure cap are made of chemically not bonding materials, the closure cap may be released from the tip connector by unscrewing the closure cap. Since the RFID chip is embedded in the tip connector and the antenna in the closure cap, the rotational motion applied to the closure cap has the effect of breaking the bridge 22, thereby inactivating the RFID chip.

As better seen in FIG. 10, in a preferred embodiment the antenna 21 comprises a plurality of protrusions 210 extending radially inwardly into the material of the closure cap. These protrusions create a rotational engagement of the antenna 21 with the closure cap 101, and avoid that the antenna be detached from the closure cap when the closure cap is unscrewed from the tip connector.

The tip cap according to the second embodiment of the invention may be fabricated as follows.

A mold adapted for two-shot injection molding of the tip connector and the closure cap is provided.

The RFID tag is placed in the mold, the antenna being located so as to be at the interface between a part of the mold defining the tip connector and a part of the mold defining the closure cap.

A first material (e.g. polycarbonate) is injected into the mold so as to form the tip connector 100, the chip and a first part of the bridge (i.e. the part of the bridge adjacent to the chip) of the RFID tag being overmolded in said first material. Then, a second material (e.g. a thermoplastic elastomer) chemically not bonding with the first material is injected into the mold so as to form the closure cap 101, the antenna 21 and a second part of the bridge (i.e. the part of the bridge adjacent to the antenna) being overmolded in said second material.

An advantage of this process is that the tip cap is directly obtained with the tip connector and the closure cap in a releasably assembled state.

Of course, the appended drawings are provided for sake of illustration only and are not intended to limit the scope of the present invention.

## Claims

1. Tip cap (1) for a medical injection device, comprising:
- a tip connector (100) configured to engage a tip of the medical injection device, comprising a main body (100a) and an end portion (100b) connected to the main body by a frangible portion (100c);
- a closure cap (101) configured to sealingly close a distal opening of the tip, the closure cap being releasably connected to the tip connector (100) by mutually engaging threaded portions (1002, 1012);
the tip cap (1) further comprising an RFID tag (2) integral with the tip connector (100) and comprising a chip (20) and an antenna (21),
wherein the chip (20) is overmolded in one of the main body (100a) and the end portion (100b) and the antenna (21) is overmolded in the other one of the main body (100a) and the end portion (100b), the chip (20) and the antenna (21) being electrically connected by a bridge (22) extending through the frangible portion (100c) of the tip connector (100), the bridge (22) being configured to break under a relative movement applied to the closure cap (101) and the tip connector (100) in order to release the closure cap from the tip connector.

2. Tip cap according to claim 1, wherein the closure cap (101) comprises a sealing septum (102) configured to abut the tip (10) of the medical injection device.

3. Tip cap according to any one of claims 1 to 2, wherein the antenna (21) presents a ring shape extending about a longitudinal axis (X) of the tip cap (1).

4. Tip cap according to claim 3, wherein the bridge (22) is parallel to the longitudinal axis (X) of the tip cap.

5. Tip cap according to claim 3, wherein the bridge (22) is orthogonal to the longitudinal axis (X) of the tip cap.

6. Tip cap according to any one of claims 1 to 5, wherein the tip connector (100) and the closure cap (101) comprise mutually engaging protrusions (1003) and notches (1013) configured to rotationally secure the end portion (100b) of the tip connector (100) to the closure cap (101), the frangible portion (100c) being configured to break under a relative rotational movement applied to the closure cap (101) and the tip connector (100) in order to release the closure cap (101) from the main body (100a) of the tip connector.

7. Medical injection device comprising a barrel and a tip (10) comprising a distal opening, and a tip cap (1) according to any one of claims 1 to 6 mounted on said tip.

8. Process for manufacturing a tip cap according to any one of claims 1 to 6, comprising:
i) providing a RFID tag (2) comprising a chip (20) and an antenna (21), the chip (20) and the antenna (21) being electrically connected by a bridge (22);
ii) placing the RFID tag in a mold adapted for injection molding of the tip connector (100);
iii) injecting at least one plastic material into the mold by using a first insert so as to form at least a part of the tip connector (100) and by overmolding the chip and the antenna, the chip and the antenna presenting a flushed surface;
iv) injecting the same plastic material as in step iii) by using a second insert and obtaining the tip connector (100) in which the RFID tag (2) is overmolded;
v) molding the closure cap (101); and
vi) releasably assembling the closure cap to the tip connector in order to obtain the tip cap.
